# EUROPEAN PATENT APPLICATION

(11) **EP 3 745 409 A1**
(43) Date of publication of application: **02.12.2020**
(21) Application number: 19177341.5
(22) Date of filing: 29.05.2019
(51) Int. Cl.: G16H 20/00

(54) **METHODS AND APPARATUS FOR GENERATING A GRAPHICAL REPRESENTATION**

(71) Applicant: Koninklijke Philips N.V., 5656 AG Eindhoven (NL)
(72) Inventor: VOS, Pieter Christiaan, 5656 AE Eindhoven (NL); HOFFMANN, Ralf Dieter, 5656 AE Eindhoven (NL); SCHUURKAMP, Gertjan Laurens, 5656 AE Eindhoven (NL)
(74) Representative: de Haan, Poul Erik

(57) **Abstract**

A computer implemented method for generating a graphical representation of a predicted effectiveness of a first treatment. The method comprises using (102) a clinical model to determine at least one indicator related to an outcome of a first treatment. An effectiveness of the first treatment is then predicted (104) based on the at least one indicator. The predicted effectiveness of the first treatment is then displayed (106) to a user, using a first graphical representation.

## Description

### FIELD OF THE INVENTION

This disclosure relates to the field of medicine. Embodiments herein relate to computer implemented methods for generating a graphical representation of a predicted effectiveness of a treatment, and systems and computer programs for the same.

### BACKGROUND OF THE INVENTION

When selecting an appropriate treatment for a patient, a medical professional (e.g. a clinician, doctor, nurse, etc.) may use various considerations to try to quantify the expected outcome of the selected treatment for the particular patient being treated. These may be patient specific such as the patient's age, their fitness for treatment or the expected improvement in quality of life following the treatment. A medical professional may also take into consideration factors specific to the disease or illness of the patient, such as the aggressiveness of the disease or the expected progression of the disease after treatment. A multitude of clinical models (or risk models) are available to support clinicians in their decision making. Examples of models may be found, for example, in the paper Lughezzani G et al. (2010) "Predictive and Prognostic Models in Radical Prostatectomy Candidates: A Critical Analysis of the Literature". Such models may provide more sophisticated indicators of outcomes of a treatment, however, in practice, the use of such models may be cumbersome, particularly if relevant models are spread over many different applications or webpages.

### SUMMARY OF THE INVENTION

As described above, although clinical models are available that can provide various indicators related to outcomes of a treatment, in practice these may be cumbersome to use, particularly if the medical professional has to interact with multiple disparate web pages, desktop applications or other application programming interfaces. Furthermore, it may be difficult to collate and compare information from different clinical models in order to select a treatment if the clinical models come from different sources in this way. There is therefore a need for improved solutions to help medical professionals collate information from different clinical models in order to enable them to select the most appropriate treatments for their patients.

Therefore, according to a first aspect, there is provided a computer implemented method for generating a graphical representation of a predicted effectiveness of a first treatment. The method comprises using a clinical model to determine at least one indicator related to an outcome of a first treatment, predicting an effectiveness of the first treatment, based on the at least one indicator, and displaying the effectiveness of the first treatment to a user using a first graphical representation.

In this way, indications from clinical models may be combined into a single predicted effectiveness and displayed in a user-friendly manner in order to improve the decision making process of a medical professional. As a result the medical professional may be able to make a more informed decision in a shorter time frame. This may provide a better user interface for selecting a treatment for a patient.

In some embodiments predicting an effectiveness of the first treatment may comprise using a machine learning model to predict the effectiveness, based on the at least one indicator.

In some embodiments the machine learning model may be trained to predict a survival outcome, a pathological outcome and/or a functional outcome of the first treatment.

In some embodiments the machine learning model may comprise an ensemble classifier.

In some embodiments predicting an effectiveness of the first treatment may comprise summing values of the at least one indicator and/or taking a weighted average of values of the at least one indicator.

In some embodiments a height, width and/or area of a first portion of the first graphical representation may be determined by the predicted effectiveness of the first treatment.

In some embodiments the first graphical representation may be divided into portions related to a survival outcome, pathological outcome and/or functional outcome of the treatment.

In some embodiments the first graphical representation may be divided into portions corresponding to values of the at least one indicator.

In some embodiments the method may further comprise predicting an effectiveness of a second treatment, and displaying the effectiveness of the second treatment to a user, using a second graphical representation.

In some embodiments one of: a height, width and/or area of a second portion of the second graphical representation may be determined by a relative value of the predicted effectiveness of the first treatment and the predicted effectiveness of the second treatment.

In some embodiments the method may further comprise: predicting an effectiveness of a second treatment, and displaying the effectiveness of the first treatment and the effectiveness of the second treatment in the first graphical representation.

In some embodiments the effectiveness of the second treatment may be represented by a second portion in the first graphical representation and the second portion may be overlain on top of the first portion.

In some embodiments the method may further comprise selecting a treatment for the patient, based on the predicted effectiveness of the first treatment and the predicted effectiveness of the second treatment.

According to a second aspect, there is provided a system for generating a graphical representation of a predicted effectiveness of a first treatment. The system comprises: a memory comprising instruction data representing a set of instructions, a user interface, and a processor configured to communicate with the memory and to execute the set of instructions. The set of instructions, when executed by the processor, cause the processor to: use a clinical model to determine at least one indicator related to an outcome of a first treatment, predict an effectiveness of the first treatment, based on the at least one indicator, and send an instruction to the user interface to cause the user interface to display the effectiveness of the first treatment using a first graphical representation.

According to a third aspect, there is provided a computer program product comprising a computer readable medium, the computer readable medium having computer readable code embodied therein, the computer readable code being configured such that, on execution by a suitable computer or processor, the computer or processor is caused to perform the method of the first aspect.

### BRIEF DESCRIPTION OF THE DRAWINGS

For a better understanding of embodiments, and to show more clearly how they may be carried into effect, reference will now be made, by way of example only, to the accompanying drawings, in which:
Fig. 1 shows a method 100 according to some embodiments;
Fig. 2 shows example first and second graphical representations according to some embodiments herein;
Fig. 3 shows another example graphical representation according to some embodiments herein;
Fig. 4 shows an example graphical representation comprising a tooltip according to some embodiments herein;
Fig. 5 shows another example graphical representation comprising a tooltip according to some embodiments herein;
Fig. 6 shows another example graphical representation comprising a tooltip according to some embodiments herein;
Fig. 7 shows an example interactive user interface for receiving user input to update patient characteristics that are input to a clinical model; and
Fig. 8 shows a schematic of an example system according to some embodiments herein.

### DETAILED DESCRIPTION

As noted above, there is provided herein improved methods, systems and computer programs for generating a graphical representation of a predicted effectiveness of a first treatment. Embodiments described herein make it easier for a medical professional to interact with and gain actionable insights from clinical models in order to better select treatments for their patients.

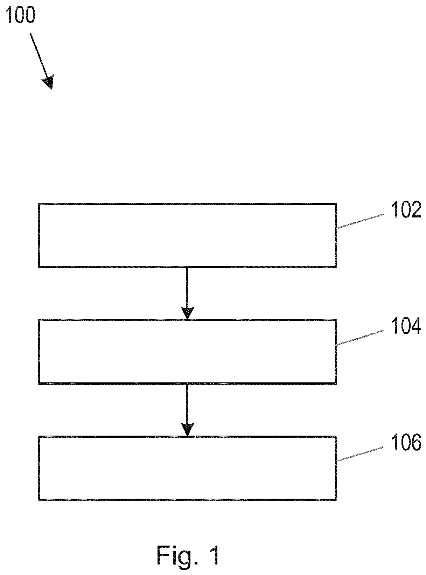

Fig. 1 illustrates a computer-implemented method 100 of generating a graphical representation of a predicted effectiveness of a first treatment. Briefly, with reference to Fig. 1, the method comprises using a clinical model to determine at least one indicator related to an outcome of a first treatment (in block 102 of Fig. 1) and predicting an effectiveness of the first treatment, based on the at least one indicator (block 104 of Fig. 1). Method 100 also comprises displaying the effectiveness of the first treatment to a user, using a first graphical representation (block 106 of Fig. 1).

By predicting an effectiveness of the first treatment, based on the at least one indicator, indicators from different clinical models may be combined into a single overall prediction of an effectiveness of the treatment. By displaying the predicted effectiveness to the user (e.g. medical professional) in a graphical format, the user is able to quickly obtain a summary of the underlying clinical models without the need to consult the individual clinical models directly (which may require different log-in details, different web pages or different desktop applications to be consulted). In this way, a medical professional's interactions with clinical models may be improved, enabling them to make more informed and quicker treatment selections for their patients.

The first treatment may comprise any prospective treatment, treatment strategy or procedure that could be performed on a patient. The treatment may relate, for example, to oncology, and may comprise, for example a cancer treatment, such as a surgical procedure to remove a tumour. In some embodiments the treatment comprises a treatment for prostate cancer. For example the first treatment may comprise one of robotic surgery, retropubic surgery, brachytherapy or external beam radiotherapy. In some embodiments the first treatment may comprise a treatment strategy such as, for example, a nerve sparing plan or pelvic lymph node dissection.

Clinical models include any model or framework that may be used to determine (e.g. predict or calculate) an indicator related to an outcome of the first treatment. For example, a clinical model may link patient characteristics to an outcome of the treatment for the patient. Clinical models may take patient characteristics such as age, height, weight of the patient as input features and output an indicator related to an outcome (e.g. a clinical outcome, or quality of life outcome) of the first treatment. Examples of clinical models include, but are not limited to, those found in the following references: Lughezzani G et al (2010) "Predictive and Prognostic Models in Radical Prostatectomy Candidates: A Critical Analysis of the Literature*";* Tosoian et al. (2017) BJU Int "Prediction of pathological stage based on clinical stage, serum prostate -specific antigen, and biopsy Gleason score: Partin Tables in the contemporary era"; Alemozaffar et al JAMA (2011) "Prediction of erectile function following treatment for prostate cancer*";* and Martini et al. BJU Int. (2018) "Development and internal validation of a side-specific, multiparametric magnetic resonance imaging-based nomogram for the prediction of extracapsular extension of prostate cancer*".* An indicator may be in the form of a score, prediction, weighting, or any other indicator that may be used to provide an indicator of an outcome of the first treatment.

In embodiments relating to prostate cancer, examples of indicators of outcomes of the first treatment include, but are not limited to, measures of potency recovery at 6, 12 or 24 months, measures of continence recovery at 1, 3 or 12 months, lymph node invasion, Gleason score, and seminal vesicle invasion. The skilled person will appreciate that these are examples only and appropriate indicators related to an outcome of the first treatment will depend on the type of treatment being considered.

In some embodiments, using (102) a clinical model to determine at least one indicator related to an outcome of a first treatment may comprise providing patient characteristics to the clinical model. The clinical model may process the patient characteristics and provide the at least one indicator based on the patient characteristics. Patient characteristics may be provided to the at least one clinical model in many ways, for example, by calling an application programming interface, API, associated with the model.

Turning now to block 104, generally, the predicted effectiveness may comprise a score, rating or ranking derived from the at least one indicator (e.g. a combined score, rating or ranking derived from the output(s) of the clinical model(s)).

In some embodiments the predicted effectiveness may relate to a survival outcome, a pathological outcome and/or a functional outcome (e.g. describing the working order of a body part) of the first treatment.

In some embodiments, predicting 104 an effectiveness of the first treatment, based on the at least one indicator may comprise combining or summarising one or more indicators into the predicted effectiveness.

For example, predicting 104 an effectiveness of the first treatment may comprise summing values of the at least one indicator and/or taking a weighted average of values of the at least one indicator. In such a way, a plurality of indicators may be combined to form a prediction of the overall efficacy of the treatment. Thus providing a combined prediction (e.g. score or ranking) for a medical professional to consider.

In some embodiments, different indicators may be grouped into different categories, for example functional, oncological or quality of life outcomes and the predicted effectiveness may be split into measures for each category. In some embodiments the number of categories and associated name of the category may be determined by the user (e.g. by receiving user input). Furthermore, in some embodiments the user may update (e.g. change) which indicators are associated with which categories. Thus, the medical professional may be able to configure the predicted effectiveness according to their needs and preferences.

In other embodiments predicting an effectiveness of the first treatment may comprise using a machine learning model to predict the effectiveness, based on the at least one indicator.

The skilled person will be familiar with machine learning and machine learning models that may be used herein. However, in brief, machine learning models comprise computer implemented mathematical models that may be trained to classify or make predictions based on a set of input features. The machine learning model takes the features as input and outputs a predicted classification or score. Herein, a machine learning model may be trained to predict the effectiveness of the first treatment based on the at least one indicator. Put another way, the outputs of clinical models may be used as input features to a machine learning model.

The skilled person will be familiar with different types of machine learning model that may be used to predict the effectiveness of the first treatment from input features comprising indicator(s) (e.g. outputs) from a clinical model. Examples of machine learning models include, but are not limited to, supervised machine learning models such as deep neural networks, logistic regression models, random forest models or ensemble classifiers. In some embodiments the machine learning model comprises an ensemble classifier.

In some embodiments, the method 100 may further comprise training the machine learning model to predict an effectiveness of the treatment from the at least one indicator. For example, the machine learning model may be trained using training data comprising indicators and ground truth outcomes (e.g. the actual effectiveness of the treatment) for previous patients that have been treated using the same treatment.

In an example embodiment, the first treatment comprises a treatment for prostate cancer, and the user comprises a urologist. In this embodiment, predicting 104 an effectiveness of the first treatment, based on the at least one indicator comprises using an ensemble classifier. The ensemble classifier uses indicators of outcomes (e.g. predictions) from clinical models that are grouped into functional and oncological outcomes. An optimal weighted average of the indicators is learnt by the ensemble classifier by training the ensemble classifier on the indicators of each clinical model (e.g. the indicators produced by the clinical models are input features for the ensemble classifier). In this embodiment, three ensemble classifiers are trained for predicting:
i) Good vs. adverse pathology outcome, where any good outcome represents good outcome of any pathology related prediction (i.e., stage, grade, positive surgical margins, positive LN's, extra prostatic extension, etc.)
ii) Good vs. poor survival outcome represented by any good/poor outcome in terms of biochemical recurrence, metastatic disease and prostate cancer death;
iii) Good vs. bad functional outcome represented by any good/bad outcome in terms of urinary or sex function

Here, survival endpoints (e.g. the ground truth outcomes of the first treatment) are defined as (classes represent increasingly worse outcomes):
class_1 in case of only biochemical recurrence;
class_2 in case of biochemical recurrence and in a later stadium metastatic disease
class_3 in case of biochemical recurrence, in a later stadium metastatic disease and the patient diseased.

Functional recovery endpoints are defined as:
class_1 in case of no adverse effect;
class_2 in case of poor urology or potency recovery;
class_3 in case of poor urology and potency recovery.

Using a machine model in this way allows a clinician to combine indicators into an overall predicted effectiveness of the treatment, reducing the complexity of multiple clinical models and multiple indications. Furthermore, the effectiveness in this sense may be defined by the training data and ground truth examples provided to the machine learning model. Thus the predicted effectiveness of the treatment may be tuned to reflect a particular medical professional's interests and preferred outcomes by defining the ground truth in the training examples.

In some embodiments the machine learning model may be trained to predict a survival outcome, a pathological outcome and/or a functional outcome of the first treatment. As such, a medical professional may be provided with different predictions allowing them to easily assess and weigh up the survival, pathological and functional prognosis associated with a treatment, This may help the medical professional to find an optimal balance between functional and oncological outcomes of a selected treatment.

Turning now to block 106, the method then comprises displaying the predicted effectiveness of the first treatment to a user, using a first graphical representation. The predicted effectiveness may be displayed using any suitable user interface (e.g. computer display screen), such as interface 806 as will be described below with respect to system 800. The predicted effectiveness may be displayed on a graphical user interface (GUI). In some embodiments the GUI may be a user interactive GUI which may be configured to receive user input from the user (e.g. mouse clicks etc).

As used herein, a graphical representation may comprise any graph, chart, or any other diagram that may be used to visually display the predicted effectiveness to the user. For example, the graphical representation may comprise a bar char, a pie chart, a scatter diagram or any other type of graph. In some embodiments, for example, the graphical representation may comprise a circular bar chart.

In some embodiments a height, width and/or area of a first portion of the first graphical representation may be determined by the predicted effectiveness of the first treatment. For example, in embodiments where the graphical representation comprises a circular bar chart, the height, width or area of a bar in the circular bar chart may positively correlate with improved predicted outcomes.

In some embodiments, the first graphical representation may be divided into portions related to a survival outcome, pathological outcome and/or functional outcome of the treatment. For example, in embodiments wherein the graphical representation comprises a circular bar chart, there may be a bar representing the survival outcome, a bar for the pathological outcome and/or a bar representing a functional outcome of the treatment.

In some embodiments, method 100 may further comprise predicting an effectiveness of a second treatment, and displaying the effectiveness of the second treatment to a user, using a second graphical representation. The second treatment may, for example, comprise an alternative or competing treatment or treatment strategy to the first treatment. The user or medical professional may need to determine which of the first and second treatments is appropriate for the patient and select an appropriate treatment from the first and second treatment.

Predicting an effectiveness of a second treatment may comprise any of the blocks outlined above for predicting an effectiveness of the first treatment, and the details of blocs 102 and 104 will be understood to apply equally to predicting the effectiveness of the second treatment.

In some embodiments, a height, width and/or area of a second bar in the second graphical representation may be determined by a relative value of the predicted effectiveness of the first treatment and the predicted effectiveness of the second treatment. For example, in embodiments where the first and second graphical representations comprise circular bar charts, the height, width or area of second bar in the second circular bar chart may be larger than the height, width or area of first bar in the first circular bar chart if the predicted effectiveness of the second treatment is greater than the predicted effectiveness of the first treatment.

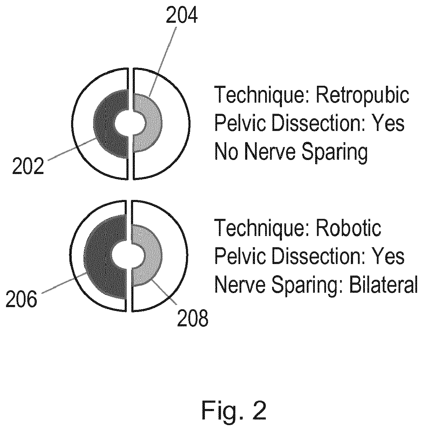

Fig.2. shows an example embodiment where the first and second graphical representations comprise circular bar charts. In this example embodiment, the predicted effectiveness of two types of surgery (e.g. two different surgical strategies) that may be used to treat a prostate cancer patient are shown side by side. Each treatment option has a different impact on quality of life and curability of the disease. The predicted effectiveness of a Retropubic procedure (e.g. first treatment) is illustrated in the upper circular bar chart and the predicted effectiveness of a Robotic procedure (e.g. second treatment) is illustrated in the lower circular bar chart. In this embodiment the predicted effectiveness comprises a predicted quality of life effectiveness and a predicted oncological effectiveness. The predicted quality of life effectiveness of the Retropubic and Robotic procedures are illustrated by bars 202 and 206 respectively and the predicted oncological effectiveness of the Retropubic and Robotic procedures are illustrated by bars 204 and 208 respectively. The two graphical representations visually embody the total quality of life impact and oncological impact of the respective treatments. In this example, the Robotic surgery (robotic with a bilateral nerve sparing surgery) has a more positive impact on quality of life, visualized as a larger portion (or bar) 206 compared to the quality of life portion 202 for the Retropubic option, as shown in Fig. 2. In this example embodiment, the predicted effectiveness measures (quality of life and oncological) are calculated by summing of all indicators relating to quality of life and oncological outcome respectively. It will be appreciated that it depends on the context of a predicted effectiveness as to whether a large circle is associated with a good outcome. In the example in Fig. 2, the quality of life portions 202, 206 should be maximal and oncological portions 204, 208 should be minimal. However, the skilled person will appreciate that this depends on how the predicted effectiveness is defined. For example, in other embodiments, a scale could be defined whereby the oncological portion should be maximised. It will be further appreciated, that the specific types of surgery are also examples and that other types of surgery and/or other types of treatment may be displayed.

In this way a medical professional may quickly gain an overview of the functional and oncological effectiveness of the two prospective treatments in order to select an optimal balance between oncological and functional outcome. Thus an appropriate treatment for a particular patient may be selected that draws on the underlying clinical models without the medical professional needing to consult the models individually. Furthermore, multiple clinical model outcomes can be compared in a single view, instead of prediction outcomes that are shown in isolation (which is typically the case for apps, websites or other applications).

Turning back now to method 100, in some embodiments, the method 100 may further comprise predicting an effectiveness of a second treatment, and displaying the effectiveness of the first treatment and the effectiveness of the second treatment in the first graphical representation. By displaying the first and second treatments in the same graphical representation, the user or medical professional may more quickly be able to compare the effectiveness of the two treatments.

In some embodiments, the effectiveness of the second treatment may be represented by a second portion in the first graphical representation and the second portion may be overlain on top of the first portion.

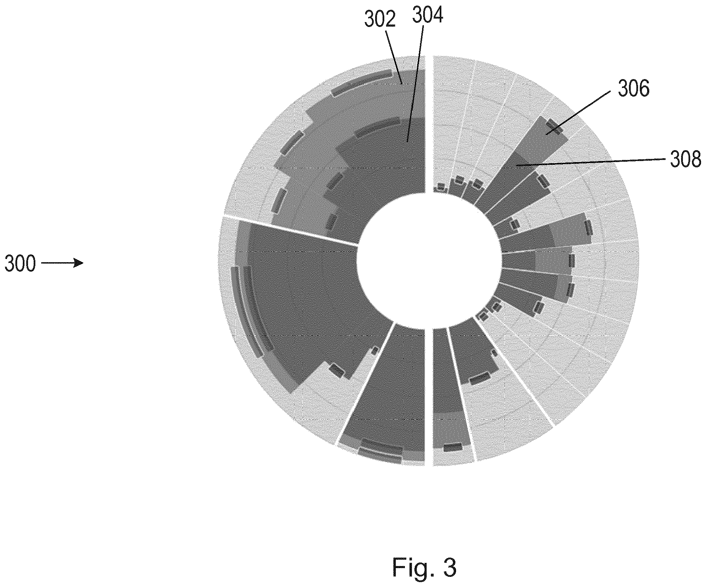

This is illustrated in Fig. 3 which shows an embodiment wherein the first graphical representation comprises a circular bar chart 300. In this embodiment, a first treatment is represented by the first portion (e.g. first bar) 302 shaded in light grey and a second treatment is represented by a second portion (e.g. second bar) 304 shaded in dark grey. In this embodiment, the first and second portions 302, 304 represent the predicted functional effectiveness of the first treatment and the predicted functional effectiveness of the second treatment respectively. The portions 302, 304 are overlain for direct comparison such that it can easily be seen that the predicted functional effectiveness of the first treatment is better than that of the second treatment.

More generally, in some embodiments, the first graphical representation may be divided (or further divided) into portions corresponding to values of the at least one indicator. This enables a medical professional to consider both the predicted effectiveness of the first treatment and the underlying indicators related to individual outcomes (e.g. clinical model outputs) when selecting a treatment for a patient.

This is also shown in the example embodiment of Fig. 3 whereby portions (e.g. bars) 306, 308 on the right hand half of the graphical representation 300 represent the indicator "localised disease" of the first and second treatments respectively.

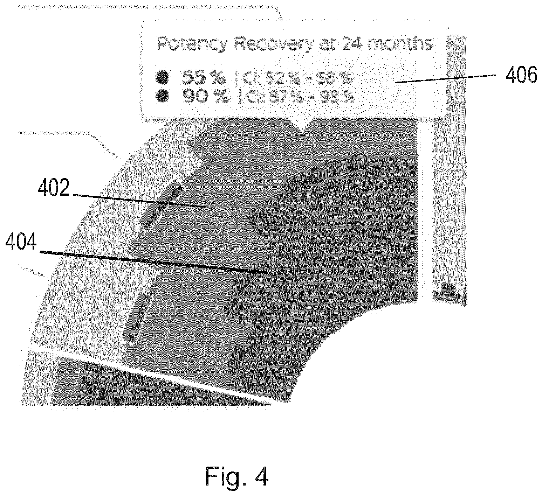

Fig. 4 illustrates a further embodiment of the method 100. In this embodiment, the graphical representation comprises a circular bar chart and the predicted effectiveness of a first and second treatment are represented by first and second portions 402, 404 respectively that are overlain on top of each other. In this embodiment, the method comprises displaying a tool tip 406 displaying confidence intervals for the predicted effectiveness. The tool tip is displayed, for example, when the user hovers over or clicks on (e.g. with a mouse or other user input) a portion of the graphical display.

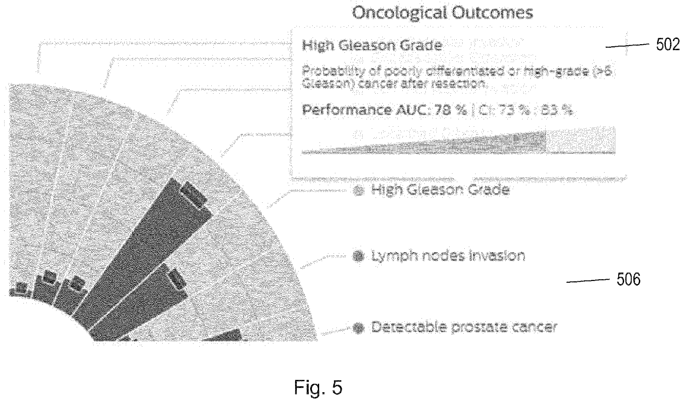

Fig. 5 illustrates a further embodiment of the method 100. In this embodiment clicking on a portion representing a predicted effectiveness shows a tooltip 502 with first level details of the underlying clinical model(s) used in block 102. These may include a description of the clinical model(s) and the discriminating performance of the clinical model(s). In this example, the indicators produced by the clinical models may be accompanied by a colour code 506, for example, green, yellow and red corresponding to a discriminating performance larger than 80% (good performance), between 70% and 80% (moderate performance) and below 70% (poor performance), respectively.

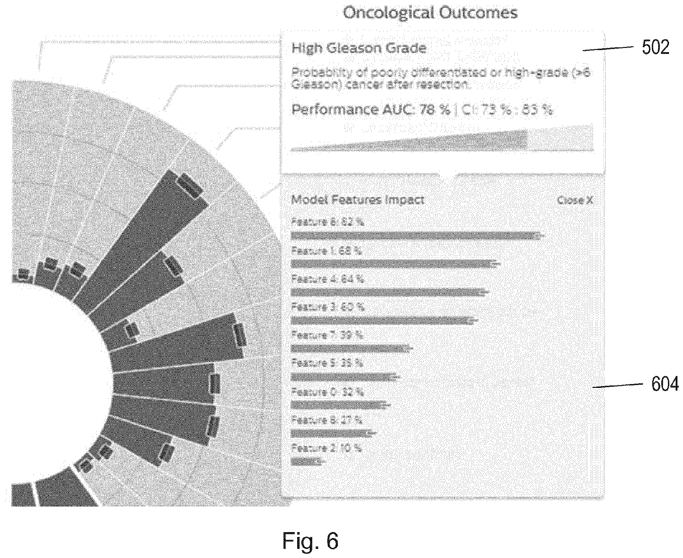

In some embodiments, and as is illustrated in Fig. 6, clicking on the first level details 502 may reveal further details 604 of the underlying clinical model(s) that provide the indicators that are used to produce the predicted effectiveness. For example, the further details may include the input patient characteristics used by the clinical models to produce the indicators and/or the relative impact each patient characteristic has on the discriminating performance of the respective clinical model.

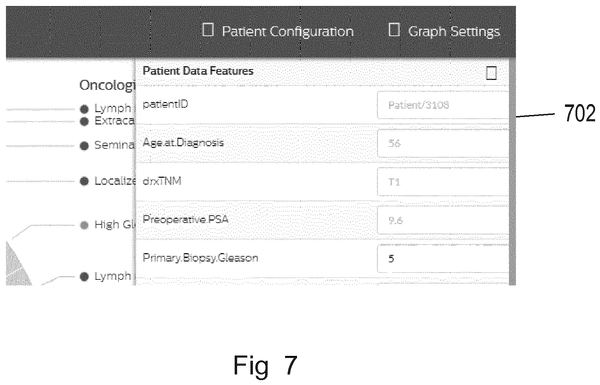

In some embodiments, a user may be able to interact with the predicted effectiveness, clinical models and the underlying patient data used for training and testing the clinical models with real-time feedback. For example, as shown in Fig. 7, in some embodiments, displaying 106 the predicted effectiveness of the first treatment to a user may comprise displaying the predicted effectiveness using a user interactive graphical user interface, GUI. In such embodiments, the user may be able to provide user input, for example to change or modify, one or more patient characteristics that are used by the clinical model(s) to determine the at least one indicator. Modifying a patient characteristic in this way may, for example, update all indicators from clinical models that take the updated patient characteristic as an input parameter. For example the user input may be obtained using a text input field, 702. The user input may be used in real time to update the at least one indicator and thus update the predicted effectiveness of the first treatment. The user (medical professional) may thus modify a patient characteristic and observe in real-time the impact on the predicted effectiveness.

In some embodiments the user may further modify the predicted effectiveness by adding and/or removing patient characteristics. The method 100 may then comprise real-time training and testing of a clinical model in order to determine an updated indicator related to an outcome of the first treatment. In this way, the user (medical professional) can determine how the predictive effectiveness is influenced by changes to the clinical models.

Turning now to other embodiments, in some embodiments the method 100 may further comprise selecting a treatment for the patient, based on the predicted effectiveness of the first treatment and the predicted effectiveness of the second treatment. For example, the method may comprise selecting a treatment from a plurality of treatment options whereby the selected treatment has the highest predicted effectiveness. The selection may be made by the computer (or for example, by the System 800 as described below), thus providing a balanced treatment recommendation based on outputs from the one or more clinical models.

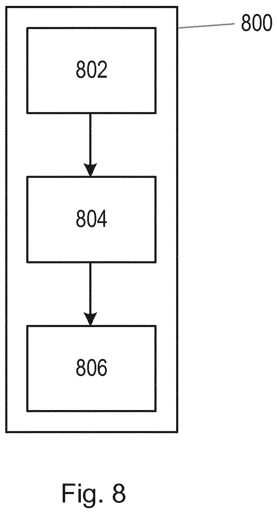

Turning now to Fig. 8, there is a system 800 configured for generating a graphical representation of a predicted effectiveness of a first treatment. The system 800 comprises a memory 804 comprising instruction data representing a set of instructions. The system 800 further comprises a processor 802 configured to communicate with the memory 804 and to execute the set of instructions. The set of instructions when executed by the processor may cause the processor to perform any of the embodiments of the method 100 as described above. The system 800 further comprises a user interface 806.

Generally, the system 800 may comprise, for example, a personal desktop computer, a laptop, a tablet computer or a mobile phone. The system 800 may also be a distributed system, for example, one or more parts may be accessible over the internet. The skilled person will appreciate however that these are only examples of the system 800 and that other configurations are possible.

In some implementations, the instruction data can comprise a plurality of software and/or hardware modules that are each configured to perform, or are for performing, individual or multiple blocks of the method described herein. In some embodiments, the memory 804 may be part of a device that also comprises one or more other components of the system 800 (for example, the processor 802 and/or one or more other components of the system 800). In alternative embodiments, the memory 804 may be part of a separate device to the other components of the system 800.

In some embodiments, the memory 804 may comprise a plurality of sub-memories, each sub-memory being capable of storing a piece of instruction data. In some embodiments where the memory 804 comprises a plurality of sub-memories, instruction data representing the set of instructions may be stored at a single sub-memory. In other embodiments where the memory 804 comprises a plurality of sub-memories, instruction data representing the set of instructions may be stored at multiple sub-memories. Thus, according to some embodiments, the instruction data representing different instructions may be stored at one or more different locations in the system 800. In some embodiments, the memory 804 may be used to store information, such as data relevant to calculations or determinations made by the processor 802 of the system 800 or from any other components of the system 800.

The processor 802 can comprise one or more processors, processing units, multi-core processors and/or modules that are configured or programmed to control the system 800 in the manner described herein. In some implementations, for example, the processor 802 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different blocks and/or different parts of a single block of the method described herein.

The system 800 further comprises a user interface (804) that may comprise a computer display, a screen or any other user interface that can be used to display information (e.g. such as the effectiveness of the first treatment, the graphical representation and/or the at least one indicator) to a user.

It will be appreciated that in some embodiments, the system may comprise other components. Examples of other components that may be comprised in system 800 include but are not limited to a user interface for inputting user information (e.g. such as a keyboard, touch-screen keyboard, mouse etc.), a communications interface for sending and/or receiving information and/or a power supply (e.g. a battery or power connection).

Briefly, the set of instructions, when executed by the processor 802, cause the processor 802 to use a clinical model to determine at least one indicator related to an outcome of a first treatment, predict an effectiveness of the first treatment, based on the at least one indicator, and send an instruction to the user interface 806 to cause the user interface to display the effectiveness of the first treatment using a first graphical representation. Using a clinical model, predicting an effectiveness and sending an instruction to a user interface were described above with respect to the method 100 and the details therein will be understood to apply equally to the operation of the system 800.

According to some embodiments, there is also a computer program product comprising a non-transitory computer readable medium, the computer readable medium having computer readable code embodied therein, the computer readable code being configured such that, on execution by a suitable computer or processor, the computer or processor is caused to perform the method 100.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer implemented method for generating a graphical representation of a predicted effectiveness of a first treatment, the method comprising:
using (102) a clinical model to determine at least one indicator related to an outcome of a first treatment;
predicting (104) an effectiveness of the first treatment, based on the at least one indicator; and
displaying (106) the predicted effectiveness of the first treatment to a user, using a first graphical representation.

2. A method as in claim 1 wherein predicting (104) an effectiveness of the first treatment comprises using a machine learning model to predict the effectiveness, based on the at least one indicator.

3. A method as in claim 2 wherein the machine learning model is trained to predict a survival outcome, a pathological outcome and/or a functional outcome of the first treatment.

4. A method as in claim 2 or 3 wherein the machine learning model comprises an ensemble classifier.

5. A method as in claim 1 wherein predicting (104) an effectiveness of the first treatment comprises summing values of the at least one indicator and/or taking a weighted average of values of the at least one indicator.

6. A method as in any one of claims 1 to 5 wherein a height, width and/or area of a first portion of the first graphical representation is determined by the predicted effectiveness of the first treatment.

7. A method as in any one of claims 1 to 5 wherein the first graphical representation is divided into portions related to a survival outcome, pathological outcome and/or functional outcome of the treatment.

8. A method as in any one of claims 1 to 5 wherein the first graphical representation is divided into portions corresponding to values of the at least one indicator.

9. A method as in any one of claims 1 to 8 further comprising:
predicting an effectiveness of a second treatment; and
displaying the effectiveness of the second treatment to a user, using a second graphical representation.

10. A method as in claim 9 wherein one of:
a height, width and/or area of a second portion of the second graphical representation is determined by a relative value of the predicted effectiveness of the first treatment and the predicted effectiveness of the second treatment.

11. A method as in any one of claims 1 to 8 further comprising:
predicting an effectiveness of a second treatment; and
displaying the effectiveness of the first treatment and the effectiveness of the second treatment in the first graphical representation.

12. A method as in claim 11 when dependent on claim 6 wherein the effectiveness of the second treatment is represented by a second portion in the first graphical representation and wherein the second portion is overlain on top of the first portion.

13. A method as in any one of claims 9 to 12 further comprising:
selecting a treatment for the patient, based on the predicted effectiveness of the first treatment and the predicted effectiveness of the second treatment.

14. A system for generating a graphical representation of a predicted effectiveness of a first treatment, the system comprising:
a memory (804) comprising instruction data representing a set of instructions;
a user interface (806); and
a processor (802) configured to communicate with the memory and to execute the set of instructions, wherein the set of instructions, when executed by the processor, cause the processor to:
use a clinical model to determine at least one indicator related to an outcome of a first treatment;
predict an effectiveness of the first treatment, based on the at least one indicator; and
send an instruction to the user interface to cause the user interface to display the effectiveness of the first treatment using a first graphical representation.

15. A computer program product comprising a computer readable medium, the computer readable medium having computer readable code embodied therein, the computer readable code being configured such that, on execution by a suitable computer or processor, the computer or processor is caused to perform the method as claimed in any one of claims 1 to 13.
